(19) Europäisches Patentamt
European Patent Office
Office européen des brevets

(11) **EP 2 057 993 A1**

(12) **EUROPEAN PATENT APPLICATION**
published in accordance with Art. 153(4) EPC

(43) Date of publication:
**13.05.2009 Bulletin 2009/20**

(51) Int Cl.:
*A61K 36/18* (2006.01)　　*A23L 1/30* (2006.01)
*A61K 8/97* (2006.01)　　*A61K 36/00* (2006.01)
*A61P 1/02* (2006.01)　　*A61P 29/00* (2006.01)
*A61Q 11/00* (2006.01)

(21) Application number: **07792761.4**

(22) Date of filing: **21.08.2007**

(86) International application number:
**PCT/JP2007/066148**

(87) International publication number:
**WO 2008/026473 (06.03.2008 Gazette 2008/10)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **31.08.2006 JP 2006236774**

(71) Applicant: ASAHI BREWERIES, Ltd.
**Sumida-ku**
**Tokyo 130-8602 (JP)**

(72) Inventors:
• **INABA, Hiroaki**
  **Osaka-shi**
  **Osaka 553-0004 (JP)**
• **HONMA, Daiki**
  **Moriya-shi**
  **Ibaraki 302-0106 (JP)**
• **TAGASHIRA, Motoyuki**
  **Moriya-shi**
  **Ibaraki 302-0106 (JP)**
• **KANDA, Tomomasa**
  **Moriya-shi**
  **Ibaraki 302-0106 (JP)**
• **AMANO, Atsuo**
  **Suita-shi**
  **Osaka 564-0061 (JP)**

(74) Representative: **Harmsen, Dirk**
  **Vossius & Partner**
  **Siebertstrasse 4**
  **81675 München (DE)**

(54) **METHOD FOR PRODUCTION OF HOP PREPARATION, HOP PREPARATION, ANTIINFLAMMATORY AGENT, FOOD/BEVERAGE, AND ORAL PRODUCT**

(57)　　A method for producing a preparation that can be used in the prevention and treatment of inflammatory diseases, including periodontitis caused by P. gingivalis.

Specifically, the invention provides a method for producing a hop preparation comprising the following steps (1) to (3):

(1) adjusting the pH of a polyphenol-containing liquid prepared from hop bracts to 6 to 7 and passing the liquid through a gel-type synthetic resin to allow components including useful substances to be adsorbed onto the resin;

(2) washing the resin obtained in the step (1) with a 30 to 60% aqueous ethanol solution to elute unwanted substances, leaving the useful substances adsorbed to the resin; and

(3) washing the resin obtained in the step (2) with a 70% or higher aqueous ethanol solution or ethanol to elute the components including the useful substances and forming the preparation from the eluted fraction.

EP 2 057 993 A1

**Description**

TECHNICAL FIELD

The present invention relates to a hop bract preparation that has anti-inflammatory effects, and a production method and use thereof.

BACKGROUND ART

Inflammation is one of the body' s defense responses to stimuli. In general, inflammation is accompanied by flare, fever, swelling, pain and dysfunction. Inflammation is involved in a wide range of diseases, including allergies, arthritis, gout, bronchitis, enterogastritis, dermatitis, psoriasis, rheumatism, Crohn's disease, Behcet's syndrome, ulcerative diseases of the digestive system, retinopathy, conjunctivitis, periodontal diseases and other diseases. Thus, an effective anti-inflammatory preparation would be of significant industrial value.

Periodontal diseases that affect periodontal tissue (i.e., gingivitis and periodontitis) are one of inflammatory diseases and each cause chronic inflammation in gingiva.

The total medical cost in Japan has now reached approximately 30 trillion yen, of which the cost for dentistry amounts to as much as 2.5 trillion yen. As proven by a recent survey conducted by the Hyogo Dental Association, Japan (Second survey on the correlation between the 8020 movement and medical cost, 2002), people having fewer lost teeth (*i.e.,* more remaining teeth) tend to require less medical costs, suggesting the increasing importance of oral hygiene in the progressively aging Japanese society. Thus, the development of effective approaches to prevent tooth loss is of significant industrial value, not only from the viewpoint of ensuring high QOL, but also from the viewpoint of suppression and reduction of medical costs.

Periodontal diseases are one of the maj or causes of tooth loss. It is almost a known fact that the diseases of periodontal tissue, or periodontal diseases, are infectious diseases caused by a variety of bacteria that form plaque within the periodontal pockets. Of these bacteria, porphyromonas gingivalis is believed to play a key role in the development of periodontal diseases.

P. gingivalis is frequently found in the periodontal pockets of patients with periodontal diseases. The bacteria produce and release vesicles (inflammation-inducing agent) containing strongly inflammatory proteinases (such as Arg-gingipain and Lys-gingipain) and lipopolysaccharides (LPS). This causes inflammation in the gingiva and other periodontal tissues, ultimately leading to the destruction of the periodontal tissue. The affected periodontal tissue can no longer support teeth and, as a result, the teeth will be lost.

A number of approaches have been proposed that use anti-inflammatory agents to prevent or ameliorate inflammatory diseases.

For example, salicylic acid derivatives, such as aspirin, and indoleacetic acid derivatives, such as indomethacin, are known to reduce inflammation by inhibiting the biosynthesis of prostaglandins. Antihistamic agents, such as diphenhydramine, are also known to reduce inflammation by decreasing the activity of histamine.

Many naturally occurring substances are also reported to exhibit anti-inflammatory activity.

According to Non-Patent Document 1, 2-[(2-methylpropanyl)-phloroglucinol]-1-O-β-D-glucopyranoside (which will be referred to simply as "MPPG, hereinafter) inhibits cyclooxygenase-1 (COX-1). However, the compound has not been reported to have any effects on cyclooxygenase-2 (COX-2). Cyclooxygenases are important enzymes involved in the synthesis of prostaglandins. Furthermore, Patent Documents 1 and 2 describe that quercetin glycosides, such as isoquercitrin (quercetin-3-O-β-D-glucopyranoside), and kaempferol derivatives, such as astragalin (kaempferol-3-O-β-D-glucopyranoside), exhibit anti-inflammatory activity.

No study has ever reported an anti-inflammatory preparation that uses hop as a starting material and contains MPPG, isoquercitrin and astragalin, all in high amounts.

Patent Document 3 discloses a column purification technique for purifying polyphenols. This technique, however, does not involve the concept of adjusting the pH during passing of liquid samples so as to control the purification of desired substances. In addition, the fraction obtained by the disclosed technique contains insignificant amounts of the useful substances: 3.1% MPPG, 3.4% isoquercitrin and 1.5% astragalin. Thus, the technique is of little usefulness.

Patent Document 4 also describes a technique for purifying polyphenols from hop. Although the technique can purify proanthocyanidin and other high-molecular weight polyphenols at higher purities than the technique described in Patent Document 3, it relies primarily on centrifugation and, like the technique of Patent Document 3, does not involve the concept of adjusting the pH during passing of liquid samples through a column to control the purification of desired substances.

Patent Document 5 describes that an ethanol extract of used hop (product remaining after $CO_2$ extraction) exhibits anti-inflammatory activity. The present inventors tried to reproduce the experiment, but it turned out that the extract was of essentially different nature from the fraction obtained by the present invention.

As described in Patent Documents 6 through 12, hop bract polyphenols, in particular high-molecular weight proanthocyanidin, are known to have various effects, including anti-decaying effect, deodorant effect, foam-stabilizing effect, anti-tumor metastasis effect, topoisomerase inhibitory effect, protein toxin-neutralizing effect and enamel decalcification inhibitory effect. To the contrary, little is known about the low-molecular weight components.

Patent Document 1 Japanese Patent Application Laid-Open No. Hei 8-133981
Patent Document 2 JapaneseTranslationNo.2004-517836ofPCT International Application
Patent Document 3 Japanese Patent No. 3477628
Patent Document 4 WO2004/052898 Pamphlet
Patent Document 5 Japanese Translation No. 2006-508182 of PCT International Application
Patent Document 6 Japanese Patent No. 3254553
Patent Document 7 Japanese Patent Application Laid-Open No. Hei 10-025232
Patent Document 8 Japanese Patent Application Laid-Open No. Hei 9-163969
Patent Document 9 Japanese Patent Application Laid-Open No. 2000-327582
Patent Document 10 Japanese Patent Application Laid-Open No. 2001-039886
Patent Document 11 WO 02/078726 Pamphlet
Patent Document 12 WO 2004/096165 Pamphlet
Non-Patent Document 1 Bohr G. et al., J. Nat. Prod., 2005; 68, 1545-1548.

DISCLOSURE OF THE INVENTION

PROBLEMS TO BE SOLVED BY THE INVENTION

In view of the above-described problems, it is an object of the present invention to provide a preparation that can be used in the prevention and treatment of inflammatory diseases, including periodontitis and gingivitis caused by P. gingivalis. It is another object of the present invention to provide a method for producing such a preparation.

MEANS FOR SOLVING THE PROBLEMS

In the course of studies to find a way to achieve the foregoing obj ects, the present inventors have found that a preparation obtained from hop bracts by the method of the present invention contains high concentrations of MPPG, isoquercitrin and astragalin and have anti-inflammatory effects, particularly against gingivitis. To obtain this preparation, a polyphenol preparation is first prepared as follows: A water-soluble fraction is extracted from hop bracts. The extract is passed through a gel-type synthetic resin and the resin is washed with water or an aqueous ethanol solution. The resin is further eluted with ethanol or an aqueous ethanol solution to obtain the polyphenol preparation (Patent Document 3). This polyphenol preparation may be separated by an ultrafiltration membrane into a high-molecular weight fraction (which does not pass through the ultrafiltration membrane) and a low-molecular weight fraction (which passes through the ultrafiltrationmembrane) (Patent Document 6). The polyphenol preparation or the low-molecular weight fraction of the polyphenol preparation is then purified by the method of the present invention to make the preparation of the present invention.

The preparation so obtained strongly inhibited inflammation induced by P. gingivalis in the immortalized cells derived from human gingival epithelium and has thus been proven effective in the prevention and treatment of inflammatory diseases, including periodontitis and gingivitis. The present inventors used the preparation in food and beverage products, mouth washes and other quasi-drugs and thereby completed the present invention.

Accordingly, the present invention concerns the following:

1) The method for producing a hop preparation comprising the following steps (1) to (3):

(1) adjusting the pH of a polyphenol-containing liquid prepared from hop bracts to 6 to 7 and passing the liquid through a gel-type synthetic resin to allow components including useful substances to be adsorbed onto the resin;
(2) washing the resin obtained in the step (1) with a 30 to 60% aqueous ethanol solution to elute unwanted substances, leaving the useful substances adsorbed to the resin; and
(3) washing the resin obtained in the step (2) with a 70% or higher aqueous ethanol solution or ethanol to elute the components including the useful substances and forming the preparation from the eluted fraction.

2) A hop preparation obtained by the method according to 1).
3) An anti-inflammatory agent containing the hop preparation according to 2).
4) The anti-inflammatory agent according to 3) for use as an anti-gingivitis agent.

5) A food and beverage product containing the hop preparation according to 2).

6) An oral product containing the hop preparation according to 2).

BEST MODE FOR CARRYING OUT THE INVENTION

It should be noted that although the preparation described in Patent Document 3 is reported to have not only antioxidant activity, but also various other effects, including anti-decaying effect, deodorant effect, foam-stabilizing effect, anti-tumor metastasis effect, topoisomerase inhibitory effect, protein toxin-neutralizing effect and enamel decalcification inhibitory effect, all of these effects are attributed to the high-molecular weight fraction of the polyphenol preparation, which is obtained by separating the polyphenol preparation by an ultrafiltration membrane into the high-molecular weight fraction (which does not pass through the ultrafiltration membrane) and a low-molecular weight fraction (which passes through the ultrafiltration membrane). Accordingly, the active component of the preparation of Patent Document 3 is different from the active component of the present invention. This makes the distinction between the preparation of Patent Document 3 and the preparation of the present invention.

Hop bracts, a material to make the preparation of the present invention, are what remains after removal of lupulin glands from the hop cone. In general, hop bracts are obtained by sieving crushed hop cones to remove lupulin glands. In recent beer brewing processes, however, hop cones are directly formed into hop pellets and used in brewing without bothering to remove unnecessary hop bracts. This eliminates the need for the sieving process to remove hop bracts. In this regard, any material that contains hop bracts can be used in the present invention, including hop cones and hop pellets.

These materials are then subjected to extraction with, for example, an aqueous alcohol solution, adsorption to a gel-type synthetic resin, washing with, for example, water, and elution with an approximately 50% aqueous ethanol solution, to give a hop bract-derived polyphenol fraction. The technique described in Patent Document 3 may suitably be used to carry out this process.

When necessary, the polyphenol fraction may further be passed through an ultrafiltration membrane. The fraction that has passed through the ultrafiltration membrane contains hop bract-derived low-molecular weight polyphenols. The technique described in Patent Document 6 may suitably be used to carry out this process. The ultrafiltration process may be omitted.

The preparation of the present invention is obtained by further purifying the hop bract-derived polyphenol fraction or the low-molecular weight fraction of the hop bract-derived polyphenol fraction.

Specifically, the hop bract-derived polyphenol fraction or the low-molecular weight fraction of hop bract-derived polyphenol fraction is prepared as a 1 to 50%, preferably 5 to 15% aqueous solution. The pH of the solution is then adjusted to 6 to 7, preferably to $6.5 \pm 0.2$. The pH of the solution may be adjusted by sodium hydroxide, potassium hydroxide, calcium hydroxide, pyridine, spermin or any other common base. When the polyphenol fraction does not readily dissolve in the solution, solvents such as methanol, ethanol, acetonitrile or acetone may be added in small amounts.

The aqueous solution is then passed through a column packed with a gel-type synthetic resin, such as hydrophilic vinyl polymer, hydroxypropylated dextran, styrene-divinylbenzene polymer or methacrylic acid polymer, preferably styrene-divinylbenzene polymer. The time over which the solution is passed through the column is determined so that the SV value is in the range of from 0.2 to 5, and preferably in the range of from 0.8 to 1.5. The SV value as used herein is a value defined by the following equation:

$$\text{SV value} = (\text{volume of solution passed (L)}) \,/\, \{(\text{volume of resin (L))} \times (\text{time over which the solution is passed (h)})\}$$

To eliminate the need to pack the column each time, the process may be carried out in a batch process in which the gel-type synthetic resin and the polyphenol-containing aqueous solution are brought into contact with each other in a reactor vessel.

Subsequently, the gel-type synthetic resin that has had useful substances adsorbed onto it is washed to elute high-molecular weight polyphenols and other unwanted components. The solvent used may be water, a 1 to 60% aqueous ethanol solution, an aqueous methanol solution having the same concentration, a 1 to 50% aqueous acetonitrile solution, or a 1 to 30% aqueous acetone solution. A 30 to 60% aqueous ethanol solution is preferably used.

The gel-type synthetic resin is then further washed with a solvent to elute the fraction containing the useful substances of MPPG, isoquercitrin and astragalin at high concentrations. The eluted fraction provides the hop preparation of the present invention. The solvent used may be a 70% or higher aqueous ethanol solution or ethanol, an aqueous methanol solution having the same concentration or methanol, a 60% or higher aqueous acetonitrile solution or acetonitrile, or a 50% or higher aqueous acetone solution or acetone. A 70 to 85% aqueous ethanol solution is preferably used.

The hop preparation so obtained contains MPPG, isoquercitrin and astragalin at high proportions and may be directly used as a liquid preparation or, when necessary, prepared as a powder preparation by spray drying, vacuum drying/solidification, freeze drying or other suitable techniques.

The preparation provided by the present invention inhibits the expression of mRNA of the gene encoding COX-2, an inflammatory mediator, and can thus be used as an effective anti-inflammatory agent. The preparation of the present invention has a wide range of applications, including the prevention and treatment of gingivitis, allergies and dermatitis. It is particularly suitable for the prevention and treatment of gingivitis.

The preparation of the present invention can be used in various confectionery products, food products and beverages that remain in the oral cavity for a relatively long period of time. It is particularly suitable for use in candies, chocolates, caramels, chewing gums and the like and may be used in mouthwashes, dentifrices and other oral products. When the hop bract-derived preparation is added to the food products and beverages and oral products, it is preferably prepared into an aqueous solution, an aqueous alcohol solution or an alcohol solution containing 1 to 20% anti-decaying material and added to the food product and beverage or the oral product to a final concentration of 1 to 5000 ppm, preferably 100 to 2000 ppm although it may be added as a powder.

The present invention will now be described with reference to examples, which are not intended to limit the scope of the invention.

Production Example 1

(Preparation of polyphenol fraction from hop bracts)

50 g of hop bracts were extracted with 1000 ml of a 40% aqueous ethanol solution at 50°C for 60 minutes under stirring. After filtration, the filtrate was concentrated under reduced pressure to a volume of 500 ml. The concentrate was passed through a column packed with 150 ml of a styrene-divinylbenzene resin (SEPABEADS 70, Mitsubishi Chemical Corporation). 500ml water was then passed through the column to wash the resin. Subsequently, 600 ml of a 50% aqueous ethanol solution were passed through the column and the eluate was collected and freeze-dried to obtain 1.7 g of a hop bract polyphenol fraction as an odorless pale yellow powder. The product had a slightly bitter taste. The yield from the hop bracts was 3.4%.

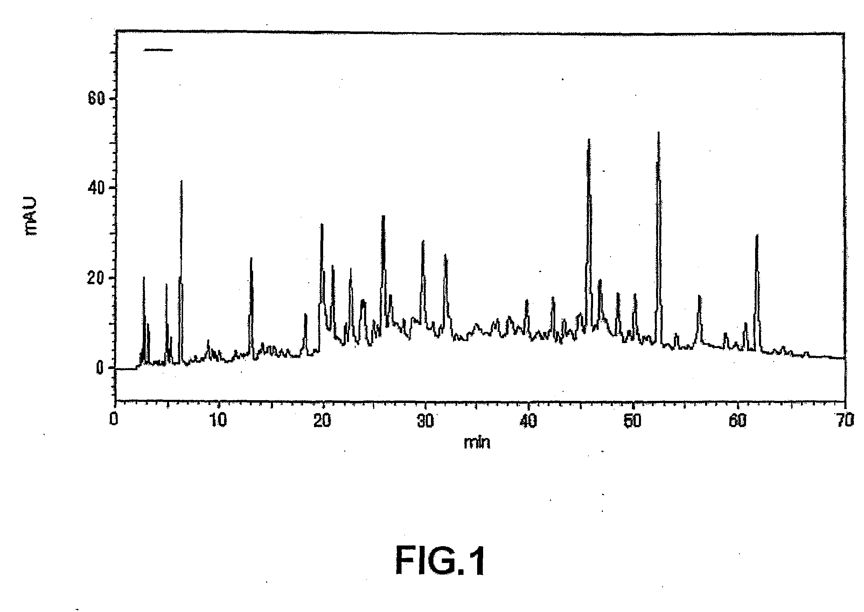

The reverse-phase HPLC chromatogram of the hop preparation obtained in Production Example 1 is shown in Fig. 1.

(Conditions for HPLC analysis)

Column: Inertsil ODS-3 (GL Sciences)
Column oven: 40°C
Flow rate: 1.0 mL/min
Detection: 280 nm
Mobile phase A: 0.1% HCOOH
Mobile phase B: 0.1% HCOOH: $CH_3CN$ = 50:50
Gradient condition: 0 → 70 min: 90% A → 40% A

Production Example 2

(Further purification of polyphenol fraction through an ultrafiltration membrane)

16.4 g of hop bract polyphenol obtained as in Production Example 1 were dissolved in 500 mL of a 50% aqueous ethanol solution. The solution was passed through an ultrafiltration membrane with a molecular weight cutoff of 10,000. The ultrafiltration gave 3.7 g of a high molecular weight fraction (the fraction that did not pass through the membrane) and 12.4 g of a low molecular weight fraction (the fraction that passed through the membrane) of the hop bract polyphenol.

[Example 1]

(Further purification of low molecular weight polyphenol fraction)

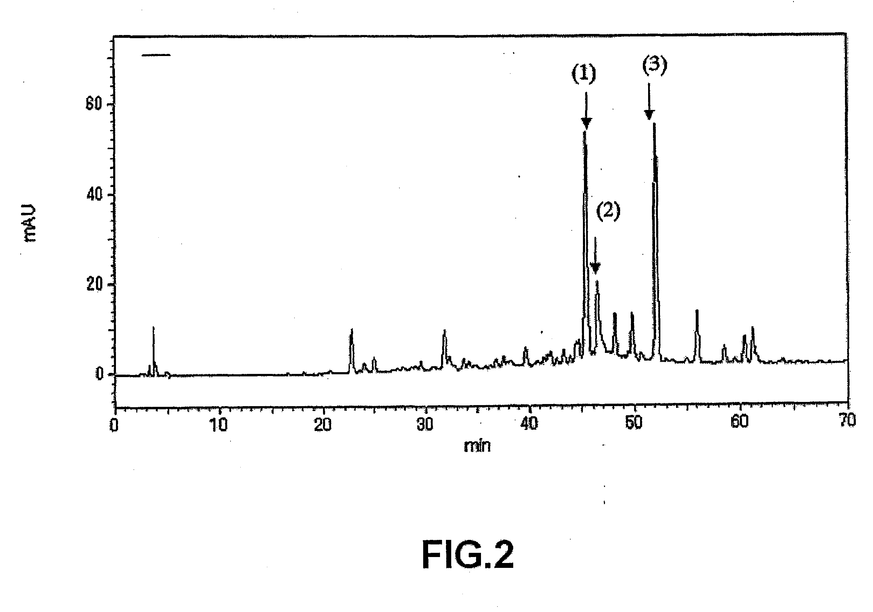

10 g of the low molecular weight fraction of the hop bract polyphenol obtained in Production Example 2 were dissolved in 70 ml of water. The pH of the solution was adjusted to 6.5 with 5N sodium hydroxide. The solution was fractionated by column chromatography on a column packed with a styrene-divinylbenzene resin (column diameter = 25 mm, column length = 360 mm, volume of resin = 180 ml). The column was sequentially eluted with water, 5% ethanol, 25% ethanol, 50% ethanol and 80% ethanol (540 ml each) and the fractions were freeze-dried to obtain powders ranging in color from yellow to brown. The weights of the resulting powders were 3.7 g, 1.6 g, 2.3 g, 2.2 g and 0.1 g, respectively. The reverse-phase HPLC chromatogram of the 80% ethanol fraction is shown in Fig. 2. The results of the structural analysis revealed that the major peaks (1), (2) and (3) observed for the fraction were MPPG, isoquercitrin and astragalin, respectively.

[Example 2]

(Suppression of the expression of inflammation marker gene)

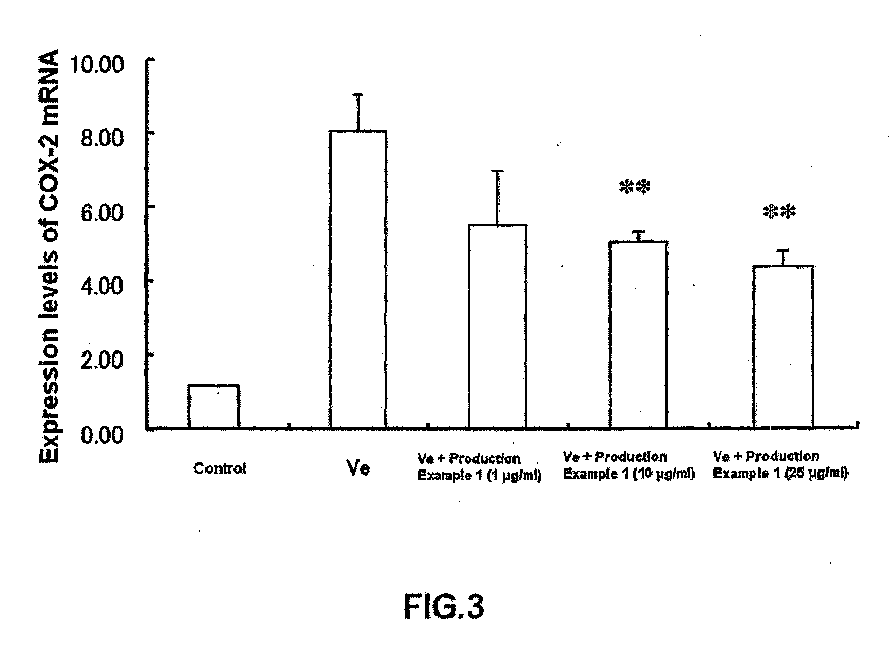

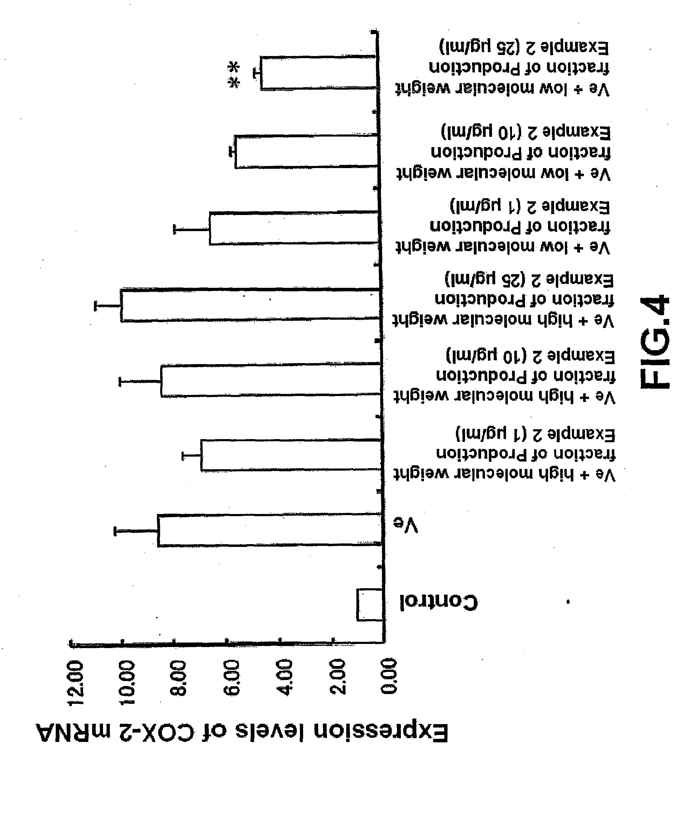

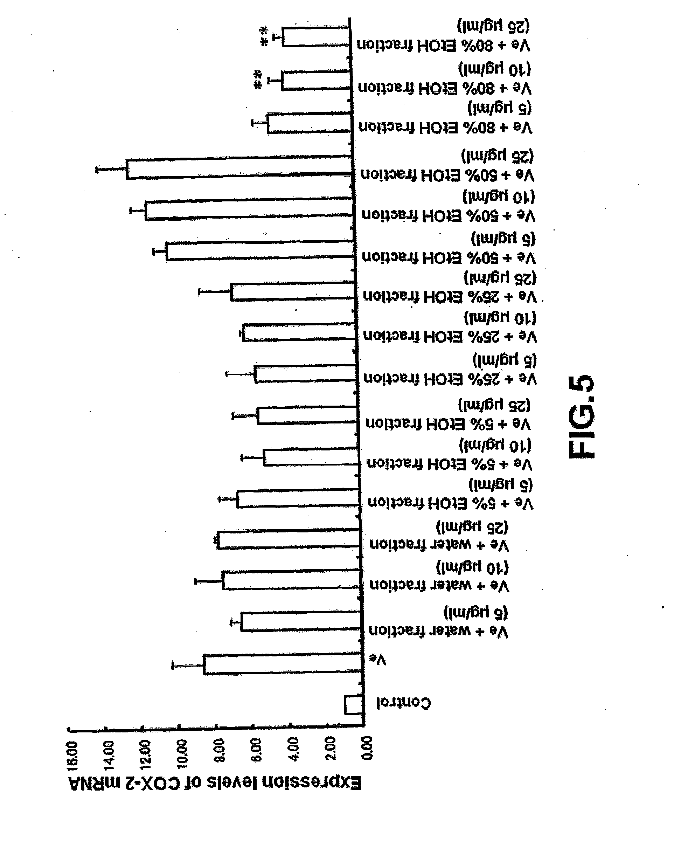

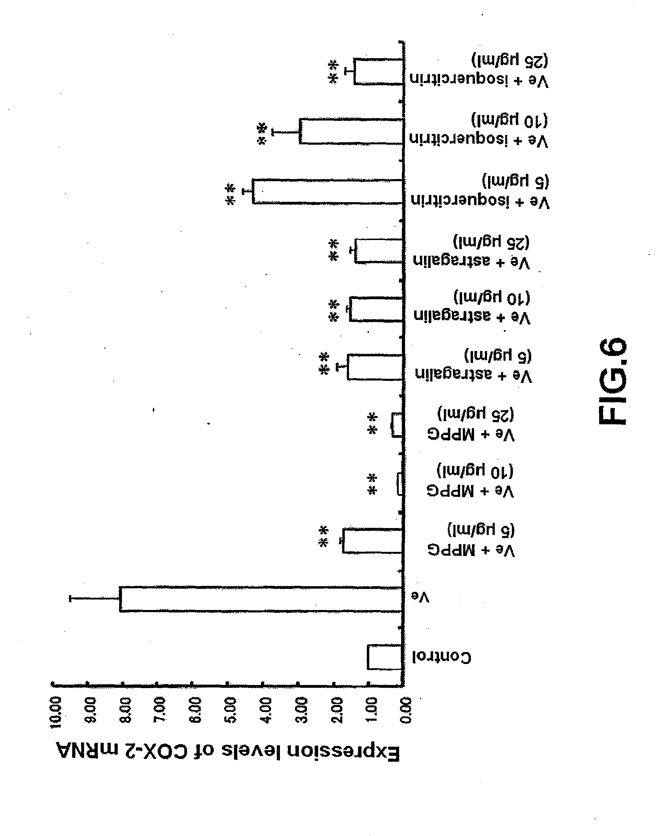

MPPG, isoquercitrin and astragalin, the major substances found in each of the high molecular weight and low molecular weight fractions of Production Examples 1 and 2, the fractions of Example 1 and the 80% ethanol fraction of Example 1, were incubated with human gingival epithelium-derived immortalized cells stimulated with the vesicles obtained from P. gingivalis (inflammation-inducing agent). The expression levels of mRNA of COX-2 gene were determined.
The P. gingivalis-derived vesicles were prepared as follows: P. gingivalis strain (ATCC 33277) was cultured for 3 days and the cells were removed from the 500ml culture by centrifugation and filtration. The supernatant was ultracentrifuged at 100,000G at 4 °C for 1 hour to obtain the desired vesicles. The expression levels of mRNA of COX-2 gene were determined by measuring the amounts of mRNA by real-time RT-PCR 24 hours after addition of each substance. The PCR was performed by using primers specific for the COX-2 gene and amplification was carried out for 45 cycles. The amount of mRNA was standardized to the amount of mRNA of glyceraldehyde-3-phosphate dehydrogenase.
The results indicate that the 80% ethanol fraction obtained in Example 1 most effectively inhibited the marker gene expression (Figs. 3 through 5). It has been demonstrated that the activity of the fraction substantially resulted from MPPG, isoquercitrin and astragalin, the major substances in the fraction (Fig. 6).
In the graphs shown, Ve indicates P. gingivalis-derived vesicles (inflammation-inducing agent) and ** indicates that the result is statistically significant relative to Ve at a significant level of 1% or less.

[Example 3]

(Identification of active components in the 80% ethanol fraction)

The 80% ethanol fraction obtained in Example 1 was fractionated by reverse-phase HPLC and the resulting fractions corresponding to the respective peaks were collected. As in Example 2, the fractions were incubated with human gingival epithelium-derived immortalized cells stimulated with the vesicles obtained from P. gingivalis (inflammation-inducing agent). The expression levels of mRNA of COX-2 were determined. The results indicate an activity for each of the three substances: MPPG, isoquercitrin and astragalin (Fig. 6).

[Example 4]

(Quantification of useful substances)

The quantification by reverse-phase HPLC of the useful substances in the 80% ethanol fraction obtained in Example 1 revealed that MPPG, isoquercitrin and astragalin were present in amounts of 14.5%, 11.2% and 7.1%, respectively. On the other hand, the fraction of Production Example 1, which was obtained by one of the disclosed methods, contained MPPG, isoquercitrin and astragalin in significantly smaller amounts of 3.1%, 3.4% and 1.5%, respectively.

[Example 5]

(Preparation of a fraction containing useful substances from the polyphenol fraction)

5 g of the hop bract polyphenol obtained in Production Example 1 were dissolved in 30ml water and the pH was adjusted to 6.7 with 1N potassium hydroxide. The solution was fractionated by column chromatography on a column packed with a styrene-divinyl benzene resin (column diameter = 25 mm, column length = 360 mm, volume of resin = 180 ml).
The column was washed with 60% ethanol and eluted with 80% ethanol (540 ml each) and the 80% ethanol fraction was freeze-dried to obtain a brown powder (0.1 g). The reverse-phase HPLC analysis of the useful substances in the 80% ethanol fraction revealed that MPPG, isoquercitrin and astragalin were present in amounts of 15.1%, 10.2% and 8.3%, respectively.

Comparative Example 1

Patent Document 4 describes that an ethanol extract of used hop (product remaining after $CO_2$ extraction) exhibits anti-inflammatory activity. The present inventors tried to reproduce the experiment, only to find that the extract was of essentially different nature from the fraction obtained by the present invention (reverse HPLC profile shown in Fig. 6). The conditions for HPLC were the same as those used in Production Example 1. The used hop product was obtained from Steiner.

The 80% ethanol fraction of Example 1 will be referred to as "preparation of the present invention," hereinafter.

[Example 6]

(Dentifrice)

| | | |
|---|---|---|
| | dibasic calcium phosphate | 42.0 |
| | glycerol | 18.0 |
| | carrageenan | 0.7 |
| | sodium lauryl sulfate | 1.2 |
| | saccharine sodium | 0.09 |
| | butyl p-hydroxybenzoate | 0.005 |
| | preparation of the present invention | 0.005 |
| | flavor | 1.0 |
| | water | 37.0 |
| | Total | 100.0 |

The components above were formulated (in weight parts given above) by a known technique to make a dentifrice.

[Example 7]

(Mouthwash)

| | | |
|---|---|---|
| | glycerol | 7.0 |
| | sorbitol | 5.0 |
| | ethanol | 15.0 |
| | sodium lauryl sulfate | 0.8 |
| | saccharine sodium | 0.1 |
| | 1-menthol | 0.05 |
| | flavor | 0.045 |
| | preparation of the present invention | 0.005 |
| | water | 72.0 |
| | Total | 100.0 |

The components above were formulated (in weight parts given above) by a known technique to make a mouthwash.

[Example 8]

(Troche)

| gum Arabic | 6.0 |
|---|---|
| magnesium stearate | 3.0 |
| glucose | 73.0 |
| lactose | 17.6 |
| dipotassium phosphate | 0.2 |
| potassium dihydrogenphosphate | 0.1 |
| flavor | 0.095 |
| preparation of the present invention | 0.005 |
| Total | 100.0 |

Using a known technique, the components above were formulated (in weight parts given above) to make a troche. Aside from the material obtained in Example 1, the materials obtained in Production Examples 2 and 3 were also used to make similar troches.

[Example 9]

(Candy)

| sucrose | 20.0 |
|---|---|
| starch syrup (75% solid content) | 70.0 |
| water | 9.5 |
| coloring agent | 0.45 |
| flavor | 0.045 |
| preparation of the present invention | 0.005 |
| Total | 100.0 |

Using a known technique, the components above were formulated (in weight parts given above) to make a candy.

[Example 10]

(Chewing gum)

| gum base | 20.0 |
|---|---|
| calcium carbonate | 2.0 |
| lactose | 77.0 |
| stevioside | 0.095 |
| preparation of the present invention | 0.005 |
| flavor | 0.9 |
| Total | 100.0 |

Using a known technique, the components above were formulated (in weight parts given above) to make a chewing gum.

[Example 11]

(juice drink)

| | |
|---|---|
| concentrated orange juice | 15.0 |
| fructose | 5.0 |
| citric acid | 0.2 |
| flavor | 0.1 |
| coloring agent | 0.15 |
| sodium ascorbate | 0.048 |
| preparation of the present invention | 0.002 |
| water | 79.5 |
| Total | 100.0 |

Using a known technique, the components above were formulated (in weight parts given above) to make a juice drink. Aside from the material obtained in Example 1, the materials obtained in Production Examples 2 and 3 were also used to make similar juice drinks.

[Example 12]

(Cookie)

| | |
|---|---|
| weak flour | 32.0 |
| whole egg | 16.0 |
| butter | 16.0 |
| sugar | 25.0 |
| water | 10.8 |
| baking powder | 0.198 |
| preparation of the present invention | 0.002 |
| Total | 100.0 |

Using a known technique, the components above were formulated (in weight parts given above) to make a cookie.

[Example 13]

(caramel)

| | |
|---|---|
| granulated sugar | 31.0 |
| starch syrup (75% solid content) | 20.0 |
| powdered milk | 40.0 |
| hydrogenated oil | 5.0 |
| salt | 0.6 |
| flavor | 0.025 |
| preparation of the present invention | 0.005 |
| water | 3.37 |
| Total | 100.0 |

Using a known technique, the components above were formulated (in weight parts given above) to make a caramel.

INDUSTRIAL APPLICABILITY

Exhibiting high anti-inflammatory activity, the hop preparation of the present invention is suitable for use as an anti-inflammatory agent or for use in food products and beverages and oral products that are expected to have anti-inflammatory effects.

BRIEF DESCRIPTION OF THE DRAWINGS

Fig. 1 is an HPLC chromatogram of a hop preparation prepared in Production Example 1.
Fig. 2 is an HPLC chromatogram of an 80% ethanol fraction prepared in Production Example 1.
Fig. 3 is a diagram showing the ability of the hop preparation of Production Example 1 to suppress the expression of mRNA of COX-2.
Fig. 4 is a diagram showing the ability of a high molecular weight fraction and a low molecular weight fraction of Production Example 2 to suppress the expression of mRNA of COX-2.
Fig. 5 is a diagram showing the ability of each fraction of Example 1 to suppress the expression of mRNA of COX-2.
Fig. 6 is a diagram showing the ability of each compound of Example 1 to suppress the expression of mRNA of COX-2.

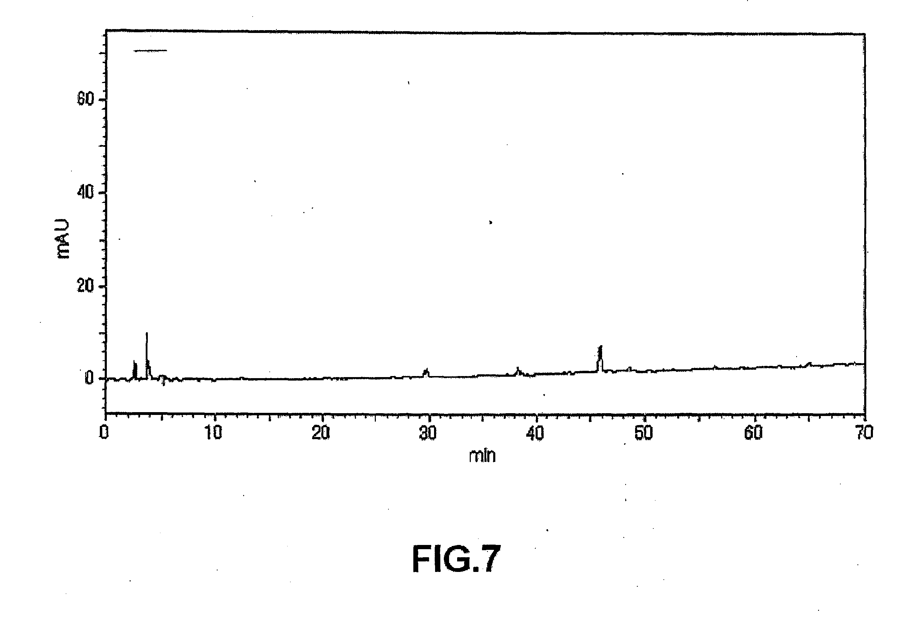

Fig. 7 is an HPLC chromatogram of an ethanol extract of used hop (product remaining after $CO_2$ extraction).

**Claims**

1. A method for producing a hop preparation comprising the following steps (1) to (3):

   (1) adjusting a pH of a polyphenol-containing liquid prepared from hop bracts to 6 to 7 and passing the liquid through a gel-type synthetic resin to allow components including useful substances to be adsorbed onto the resin;
   (2) washing the resin obtained in the step (1) with a 30 to 60% aqueous ethanol solution to elute unwanted substances, leaving the useful substances adsorbed to the resin; and
   (3) washing the resin obtained in the step (2) with a 70% or higher aqueous ethanol solution or ethanol to elute the components including the useful substances and forming the preparation from the eluted fraction.

2. A hop preparation obtained by the method according to claim 1.

3. An anti-inflammatory agent containing the hop preparation according to claim 2.

4. The anti-inflammatory agent according to claim 3 for use as an anti-gingivitis agent.

5. A food and beverage product containing the hop preparation according to claim 2.

6. An oral product containing the hop preparation according to claim 2.

FIG.1

**FIG.2**

EP 2 057 993 A1

FIG.3

**Expression levels of COX-2 mRNA**

Control

Ve

Ve + high molecular weight fraction of Production Example 2 (1 µg/ml)

Ve + high molecular weight fraction of Production Example 2 (10 µg/ml)

Ve + high molecular weight fraction of Production Example 2 (25 µg/ml)

Ve + low molecular weight fraction of Production Example 2 (1 µg/ml)

Ve + low molecular weight fraction of Production Example 2 (10 µg/ml)

Ve + low molecular weight fraction of Production Example 2 (25 µg/ml)

0.00  2.00  4.00  6.00  8.00  10.00  12.00

**

**FIG.4**

FIG.5

FIG.6

FIG.7

## INTERNATIONAL SEARCH REPORT

| International application No. |
| --- |
| PCT/JP2007/066148 |

A. CLASSIFICATION OF SUBJECT MATTER
*A61K36/18*(2006.01)i, *A23L1/30*(2006.01)i, *A61K8/97*(2006.01)i, *A61K36/00*
(2006.01)i, *A61P1/02*(2006.01)i, *A61P29/00*(2006.01)i, *A61Q11/00*(2006.01)i

According to International Patent Classification (IPC) or to both national classification and IPC

B. FIELDS SEARCHED

Minimum documentation searched (classification system followed by classification symbols)
A61K36/18, A23L1/30, A61K8/97, A61K36/00, A61P1/02, A61P29/00, A61Q11/00

Documentation searched other than minimum documentation to the extent that such documents are included in the fields searched
Jitsuyo Shinan Koho        1922-1996    Jitsuyo Shinan Toroku Koho    1996-2007
Kokai Jitsuyo Shinan Koho   1971-2007   Toroku Jitsuyo Shinan Koho    1994-2007

Electronic data base consulted during the international search (name of data base and, where practicable, search terms used)
BIOSIS/MEDLINE/WPIDS(STN), CAplus(STN), JMEDPlus(JDream2),
JSTPlus(JDream2)

C. DOCUMENTS CONSIDERED TO BE RELEVANT

| Category* | Citation of document, with indication, where appropriate, of the relevant passages | Relevant to claim No. |
| --- | --- | --- |
| X | JP 9-2917 A  (Asahi Breweries, Ltd.), 07 January, 1997 (07.01.97), Examples 1, 2, 3 & JP 3477628 B | 1-6 |
| Y | JP 2002-220340 A  (Ito En. Ltd.), 09 August, 2002 (09.08.02), Par. Nos. [0005], [0012] (Family: none) | 1-6 |
| Y | BOHR Gregor et al, Anti-inflammatory Acylphloroglucinol Derivatives from Hops (Humulus lupulus), J Nat Prod ,2005, Vol.68, No.10,Page.1545-1548 | 1-6 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>20 September, 2007 (20.09.07) | Date of mailing of the international search report<br>02 October, 2007 (02.10.07) |
| --- | --- |
| Name and mailing address of the ISA/<br>Japanese Patent Office | Authorized officer |
| Facsimile No. | Telephone No. |

Form PCT/ISA/210 (second sheet) (April 2005)

18

**INTERNATIONAL SEARCH REPORT**

| International application No. |
| --- |
| PCT/JP2007/066148 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | LEE S J, Antiinflammatory Activity of Naturally Occurring Flavone and Flavonol Glycosides, Arch Pharmacal Res.,1993, Vol.16 No.1 Page.25-28 | 1-6 |
| Y | JP 6-183940 A (Lion Corp.), 05 July, 1994 (05.07.94), (Family: none) | 1-6 |
| Y | WO 2005/53719 A2 (Indena, S.p.A.), 16 June, 2005 (16.06.05), & JP 2007-512268 A        & EP 1687015 A2 & WO 2005/53716 A3        & AU 2004294688 A1 & CN 1886147 A        & KR 200707252 A & US 2007/4661 A1        & NO 2006002355 A & IN 2006 DN02888 A | 1-6 |
| P,A | WO 2006/93202 A (Sapporo Breweries Ltd.), 08 September, 2006 (08.09.06), (Family: none) | 1-6 |

Form PCT/ISA/210 (continuation of second sheet) (April 2005)

**REFERENCES CITED IN THE DESCRIPTION**

**Patent documents cited in the description**

- JP HEI8133981 B
- JP 3477628 B
- WO 2004052898 A
- JP 2006508182 W
- JP 3254553 B
- JP HEI10025232 B
- JP HEI9163969 B
- JP 2000327582 A
- JP 2001039886 A
- WO 02078726 A
- WO 2004096165 A

**Non-patent literature cited in the description**

- **BOHR G. et al.** *J. Nat. Prod.,* 2005, vol. 68, 1545-1548